# FASCICULE DE BREVET EUROPEEN

(11) **EP 0 963 740 B1**
(45) Date de publication et mention de la délivrance du brevet: **08.01.2003**
(21) Numéro de dépôt: 99420098.8
(22) Date de dépôt: 20.04.1999
(51) Int. Cl.: A61F 2/34

(54) **Cotyle pour prothese de hanche**
Gelenkpfanne für Hüftprothese
Acetabular cup for hip prosthesis

(30) Priorité: 09.06.1998 FR 9807387
(43) Date de publication de la demande: 15.12.1999
(73) Titulaire: Biomet Merck France, 26000 Valence (FR); Cuinet, Patrick, 26800 Etoile Sur Rhone (FR); Alvernhe, Charles, 54000 Nancy (FR); Cartier, Jean-Loup, 05000 Gap (FR); Fresard, Pierre-Luc, 42100 Saint Etienne (FR); Mironneau, Antoine, 07430 Davezieux (FR); Rosas, Marc-Henri, 49100 Angers (FR); Lantuejoul Jean-Pierre, 38330 Saint Ismier (FR); Grobert, Jean-François, 71450 Blanzy (FR)
(72) Inventeur: Cuinet, Patrick, 26800 Etoile sur Rhone (FR); Alvernhe, Charles, 54000 Nancy (FR); Cartier, Jean-Loup, 05000 Gap (FR); Fresard, Pierre-Luc, 42100 Saint Etienne (FR); Mironneau, Antoine, 07430 Davezieux (FR); Rosas, Marc-Henri, 49100 Angers (FR); Lantuejoul, Jean-Pierre, 38330 Saint Ismier (FR); Grobert, Jean-François, 71450 Blanzy (FR); De Witte, Gérard, 26300 Chateauneuf sur Isère (FR); Benedetto, Muriel, 26000 Valence (FR)
(74) Mandataire: Vuillermoz, Bruno

(56) Documents cités:
- WO-A-98/15240
- DE-A- 2 246 940
- FR-A- 2 686 502

## Description

L'invention concerne un cotyle pour prothèse totale de hanche.

De manière connue, une prothèse de hanche comprend fondamentalement deux parties, respectivement un élément fémoral ou tige, destiné à être inséré dans le canal médullaire du fémur à prothéser et, portant à son extrémité libre une tête sphérique, et, un cotyle mis en place au niveau de la cavité cotyloïdienne de l'aile iliaque de l'articulation considérée et destinée à recevoir la tête sphérique dudit élément fémoral.

Plus précisément, ce cotyle est réalisé en deux parties distinctes, à savoir respectivement :
- une cupule dite de "soutien", destinée à être insérée dans la cavité cotyloïdienne de l'aile iliaque ;
- et un noyau ou insert dit "de frottement", destiné à s'insérer dans la cupule de soutien et, dont la face interne de forme hémisphérique est destinée à recevoir la tête sphérique de l'élément fémoral.

Pendant très longtemps, l'insert était réalisé de telle sorte qu'il s'insère étroitement dans la cupule de soutien, de telle sorte à coapter le plus possible avec celle-ci et donc d'être parfaitement immobile par rapport à ladite cupule.

Néanmoins, il y a une vingtaine d'année, il a été proposé une nouvelle approche qui consistait à conférer à l'insert une complète mobilité dans la cupule. De la sorte, la prothèse ainsi réalisée présente un double interface respectivement cotyle-insert et insert-tête fémoral, permettant de réduire outre les risques de luxation, tout particulièrement les risques de descellement.

En effet, par le biais de ce double interface, les ancrages primaires et secondaires sont beaucoup moins sollicités. Ainsi, lors des mouvements normaux, la tête fémorale se déplace par rapport à l'insert, ce dernier étant avantageusement autorétentif, et dans les moments extrêmes, l'insert est susceptible de se déplacer par rapport au cotyle, limitant ainsi drastiquement les risques d'arrachement et de descellement.

Par ailleurs, grâce à ce double interface de friction, l'usure du matériau constitutif de l'insert, généralement du polyéthylène est limitée, et la concentration des contraintes au niveau de l'interface os - cupule est réduite.

Si incontestablement, cette double mobilité générant ainsi un double interface de friction a permis la réalisation de progrès importants en terme de pérennité de la prothèse, le système proposé présente néanmoins certains inconvénients.

Tout d'abord, afin de permettre la mobilité de l'insert à l'intérieur de la cupule, il est nécessaire de prévoir l'autorétention de celui-ci au sein de la cupule de sorte que celle-ci présente nécessairement une forme prolongeant le simple hémisphère. Cependant, compte tenu des contraintes liées à l'anatomie, la base de la cupule n'est pas située dans un même plan, mais présente au contraire deux plans, sensiblement sécants au niveau du diamètre passant par l'axe de révolution de ladite cupule. Cette zone d'intersection génère des "cornes" saillantes, qui sont situées de façon telle qu'elles génèrent une irritation du psoas car celui-ci vient frotter à ce niveau. Cette irritation du psoas entraîne des douleurs pour le patient.

Par ailleurs, la troncature opérée au niveau de la base de ladite cupule, en raison des contraintes anatomiques peut générer des frottements entre le col de l'élément fémoral et le bord extrême du cotyle lors des mouvements de flexion-rotation. Ces frottements sont susceptibles d'engendrer une usure prématurée de la cupule et des libérations de particules nocives.

Enfin, lorsque la fixation de la cupule est complétée par des plots d'ancrage, un plot en ischion et un plot en ilion, il existe des risques de conflit entre l'insert cotyloïdien et l'extrémité supérieure desdits plots. Outre, l'usure en résultant, susceptible de libérer des particules de polyéthylène, et partant d'aboutir à une ostéolyse et donc à un descellement du cotyle, ce conflit peut constituer un obstacle à la libre rotation de l'insert au sein de la cupule et partant, peut conduire à l'annihilation de l'un des interfaces de friction, objet de la prothèse.

Une cotyle pour prothèse de hanche comprenant les caractéristiques du preámbule de la revendication 1 est connue du document FR-A-2 686 502.

L'objet de l'invention est de s'affranchir de ces différents inconvénients. Elle vise un cotyle pour prothèse de hanche, susceptible d'être mis en place selon la technologie "press-fit", c'est-à-dire une méthode de pause dans laquelle après fraisage de la cavité cotyloïdienne, le cotyle vient s'adapter directement sans système de fixation supplémentaire, et conférant la double mobilité respectivement de l'insert au sein de la cupule et de la tête de l'élément fémoral au sein de l'insert, et donc de proposer un double niveau d'interface de friction.

Ce cotyle pour prothèse de hanche comprend :
- une cupule de soutien, destinée à être mise en place dans la cavité cotyloïdienne de l'aile iliaque, et présentant une découpe partielle à partir de sa base ;
- un insert de frottement, destiné :
   - à être inséré dans la cupule de soutien tout en étant susceptible d'une complète mobilité au sein de celle-ci ;
   - et à recevoir la tête de l'élément fémoral de ladite prothèse de manière autorétentive.

Ce cotyle se caractérise en ce que la cupule est de forme cylindro-hémisphérique et se prolonge au niveau de son équateur par une portion sensiblement cylindrique, la découpe partielle qu'elle présente étant évolutive et s'étendant symétriquement d'une zone située en amont de l'axe de révolution de la cupule jusqu'à son bord inférieur.

En d'autres termes, l'invention consiste d'une part, à optimiser l'échancrure, c'est-à-dire la troncature partant de la base de la cupule tout en conférant à la découpe en résultant une forme évolutive exempte de tout angle ou de toute forme prononcée. Cette optimisation de l'échancrure est rendue possible de part la forme cylindro-hémisphérique conférée à la cupule. De la sorte, on réduit sinon annule le frottement avec le psoas, tout en permettant un positionnement correct de la cupule, et notamment sans engendrer de débordement de la cavité osseuse qui la reçoit.

Selon l'invention, le départ de la découpe évolutive est situé à quinze millimètres de l'axe de révolution de la cupule en direction de son coté supérieur, et ce, quelle que soit la taille de la cupule mise en place.

Selon l'invention, la surface externe de la cupule présente une zone en relief s'étendant sensiblement sur le tiers de sa hauteur à compter de la base, cette zone étant munie d'une pluralité de zones lisses exemptes de tout relief.

Ces zones lisses sont au moins au nombre de trois, à savoir, l'une située selon le plan de symétrie de la cupule à son extrémité supérieure, et les deux autres situées de part et d'autre symétriquement par rapport à la première.

La manière dont l'invention peut être réalisée et les avantages qui en découlent ressortiront mieux des exemples de réalisations qui suivent, donnés à titre indicatif et non limitatif à l'appui des figures annexées.

La figure 1 est une représentation schématique en perspective de la prothèse totale de hanche conforme à l'invention.

La figure 2 en est une vue en section dans le plan frontal, et la figure 3 une vue dans le plan sagittal.

La figure 4 est une vue schématique en perspective de la cupule du cotyle de l'invention.

La figure 5 en est une vue en section sagittale.

La figure 6 en est une vue frontale, tête en bas, dont la figure 7 est une vue de détail.

La figure 8 est une vue sagittale et la figure 9 est une vue du dessus de cette cupule.

La figure 10 est une représentation schématique en section de la cupule munie de l'insert et de sa coopération avec la tête fémorale.

La figure 11 est une vue de détail de la figure 10.

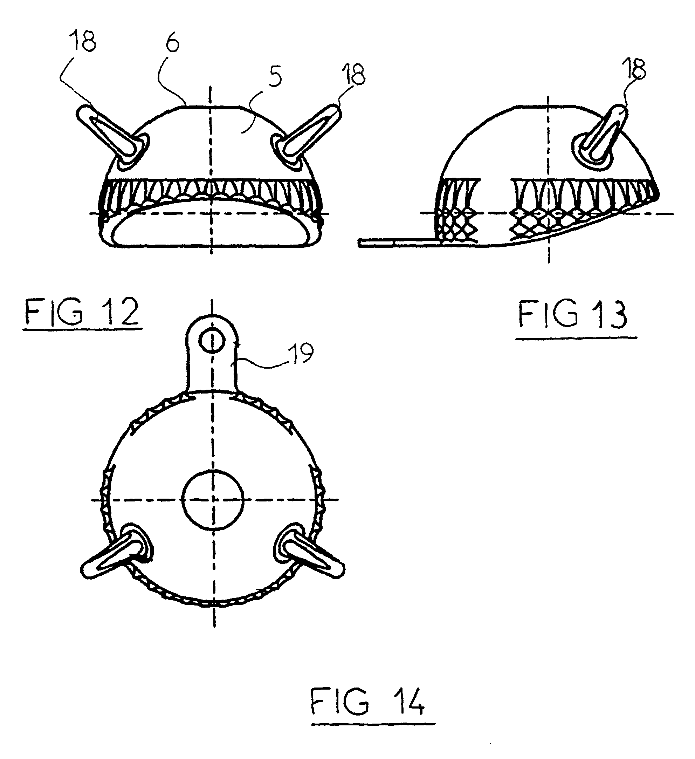

Les figures 12, 13 et 14 sont des vues similaires d'une autre forme de réalisation de la cupule conforme à l'invention, analogues aux figures 6, 8 et 9.

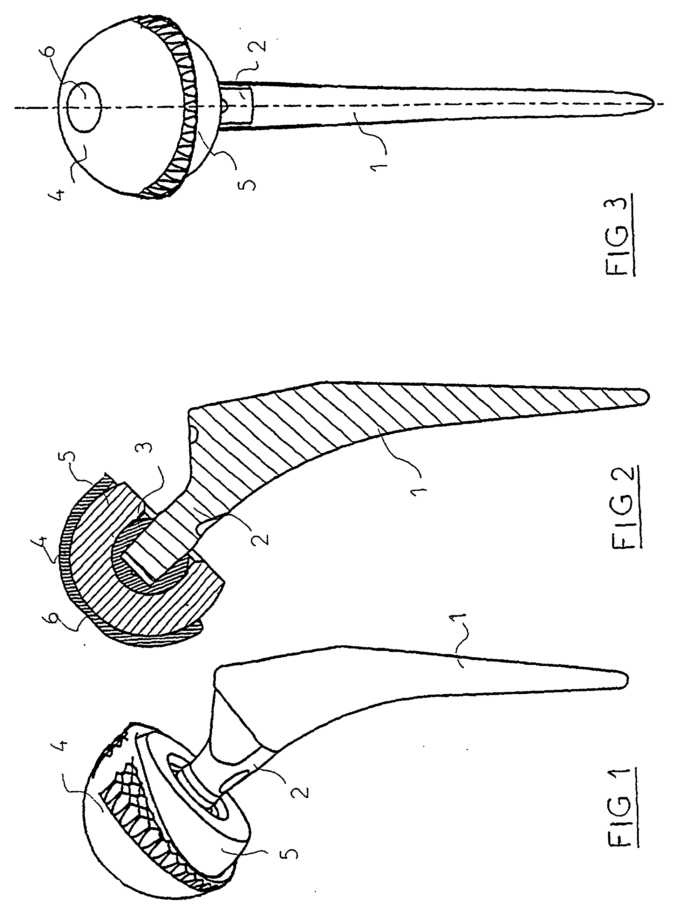

On a représenté sur les figures 1 à 3 une prothèse totale de hanche conforme à l'invention. Celle-ci comprend fondamentalement une tige (1) ou élément fémoral, destiné, comme déjà dit, à être enfoui au sein du canal médullaire du fémur à prothéser. Cette tige se prolonge par un col prothétique (2) se terminant par un cône morse, destiné à recevoir une tête sphérique (3), par exemple réalisée en céramique de zircone, en acier inoxydable, voire en alliage chrome-cobalt. Ces éléments (1, 2 et 3) constituent donc l'élément fémoral de la prothèse.

La tête sphérique (3) est destinée à être reçue dans un insert (5), notamment réalisé en polyéthylène, de surface interne complémentaire à la forme sphérique de la tête fémorale (3). Cet insert (5), présentant une symétrie de révolution, est lui-même reçu dans une cupule métallique (4). Cette dernière présente un dôme plat (6).

L'ensemble (4, 5) constitue le cotyle, cet ensemble étant destiné à être mis en place au sein de la cavité cotyloïdienne de l'articulation considérée.

L'insert de frottement (5), comme déjà dit réalisé en polyéthylène, est autorétentif de sorte que la tête (3) de l'élément fémoral est difficilement escamotable hors de celui-ci, la force d'impact étant par exemple de 140 kg. Par ce biais, on limite ainsi les risques de luxation. En revanche, elle est parfaitement mobile au sein de cet insert.

Selon l'invention, l'insert (5) est également mobile à l'intérieur de la cupule (4). De la sorte, on aboutit à une double mobilité d'une part, de l'insert par rapport à la cupule (4) et d'autre part, de la tête fémorale (3) par rapport à l'insert (5).

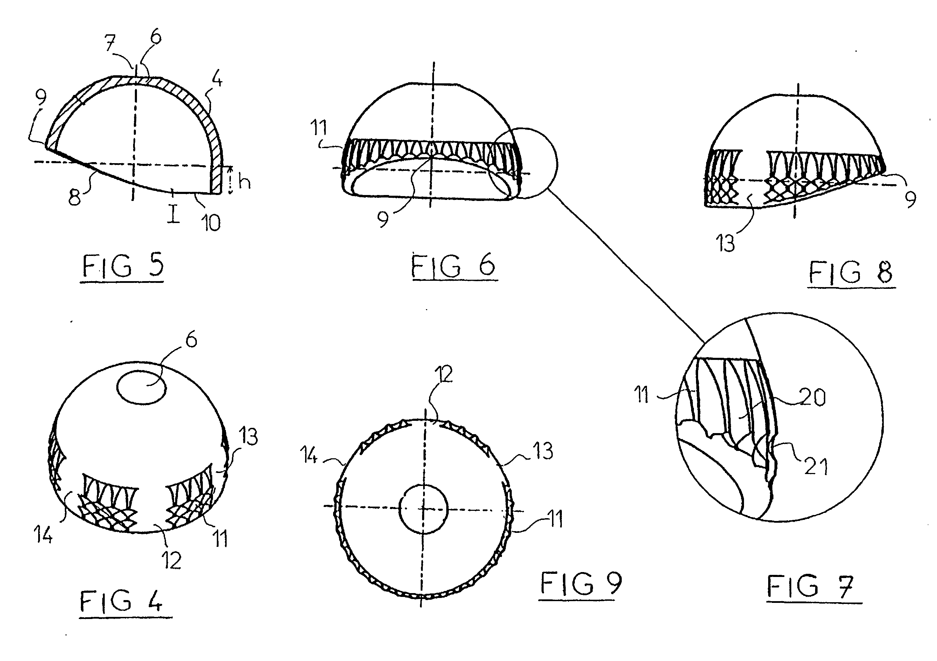

On a décrit plus en détail en liaison avec les figures 5 à 9 la cupule métallique (4) conforme à l'invention. Selon celle-ci, elle est de forme cylindrico-hémisphérique, tel qu'on peut bien l'observer dans la figure 5, en section.

On observe en effet, que la forme hémisphérique se prolonge par une portion cylindrique de hauteur h, typiquement de 8 mm quelle que soit la taille de la prothèse mise en place. En outre, la base (10) de la cupule présente une découpe évolutive (8) sur les côtés postérieurs et antérieurs, cette découpe démarrant à une certaine distance de l'axe de révolution (7) du cotyle, et notamment à 15 mm (I) en direction du coté supérieur ou proximal, pour venir rejoindre l'extrémité inférieure ou distale (9) de la cupule. Cette découpe de type "spline" permet de la sorte de limiter le débordement de la cupule par rapport à la cavité osseuse et partant, de réduire les risques de conflit et notamment de frottement avec le psoas, ces conflits engendrant comme déjà dit des douleurs pour le patient. Par ailleurs et surtout, cette découpe permet de limiter les conflits entre le col de la tige fémorale et la cupule.

Selon une autre caractéristique de l'invention, au moins le tiers à compter de la base (10) de la cupule présente sur sa face externe des reliefs (11), destinés à favoriser l'ancrage primaire, notamment lors de mise en place par la technologie press-fit.

Plus précisément, et telle que représentée dans la vue de détail de la figure 7, cette structure en relief (11) présente une surépaisseur progressive (20) constituée de saillies radiales, destinée à éviter une discontinuité dans la répartition des contraintes à l'interface structure d'accroche- sphère vierge ou lisse. Par ailleurs, ces saillies radiales permettent de limiter la rotation du cotyle une fois implanté.

Par ailleurs, cette surépaisseur progressive présente une certaine discontinuité (21), destinée à favoriser la fixation secondaire du cotyle un fois implanté.

Enfin, de par la structure générale du cotyle, les risques de protrusion sont également réduits.

Selon une autre caractéristique de l'invention, cette zone de relief est discontinue au niveau de trois zones d'ajourements différentes (12, 13, et 14), ces zones d'ajourement ainsi que représentées en figure 9 respectant l'intégrité équatoriale du cotyle osseux afin d'empêcher les micros-mouvements rotationnels et partant, tout risque de descellement du cotyle.

Ces zones d'ajourement positionnées respectivement en zone supérieure de la cupule selon son plan de symétrie et également de part et d'autre, ont également pour fonction d'affiner l'orientation de la cupule après son implantation dans la cavité osseuse car elles offrent une certaine liberté de mouvement après l'impaction, ce que ne permettrait pas d'obtenir une structure continue.

On a représenté en liaison avec les figures (12, 13 et 14) une autre forme de représentation de la cupule, dans laquelle des plots de fixation (18) dans l'ischion et le pubis viennent compléter et optimiser la fixation de ladite cupule. En d'autres termes, la fixation press-fit est renforcée par une fixation trois points à savoir d'une part, par les deux plots (18) en question, et d'autre part, par une patte supérieure (19) destinée à être vissée dans l'aile iliaque.

Avantageusement, toute la surface externe de la cupule est revêtue d'une couche d'hydroxyapatite afin de favoriser la fixation secondaire et ce, de manière connue.

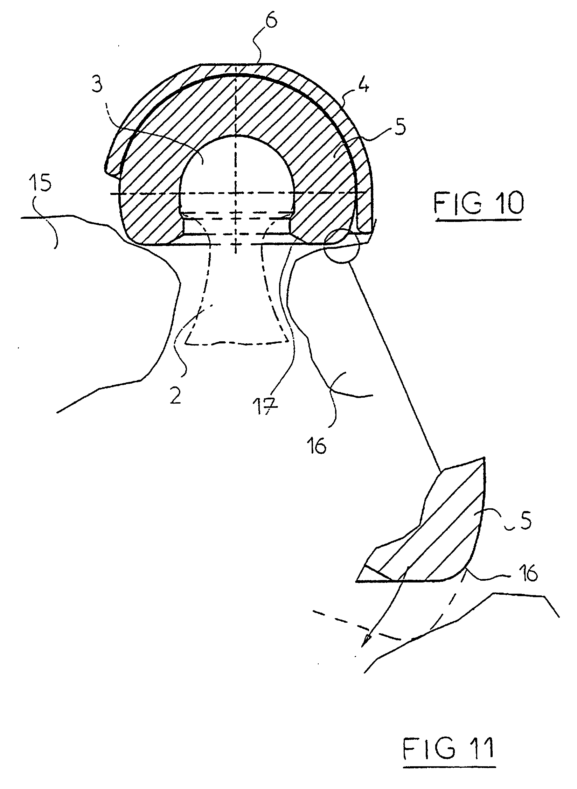

Selon une autre caractéristique de l'invention, l'arête périphérique externe (16) de l'insert polyéthylène (5) est rayonnée, ainsi qu'on peut l'observer sur les figures 10 et 11. De la sorte, cette forme particulière permet audit insert de glisser sur les tissus périarticulaires (15) qui l'entourent, lui conférant une mobilité accrue par rapport à ces tissus et annulant les phénomènes de butée que l'on rencontre avec les inserts de l'art antérieur. On diminue ainsi les frottements et les douleurs en résultant.

Parallèlement, l'arête interne (17) dudit insert (5) est chanfreinée ainsi qu'on peut bien l'observer sur la figure 10, de telle sorte à augmenter le débattement articulaire, et en outre à limiter le contact entre le col (2) de l'élément fémoral et l'insert (5), de telle sorte à limiter les risques de production de débris d'usure de polyéthylène et partant, les risque d'ostéolyse, conduisant, de manière connue, à des descellements.

Le cotyle, selon l'invention, permet donc d'optimiser le principe de la double-mobilité et du double niveau d'interface de friction, dont les avantages sont déjà connus.

De part sa structure particulière, il permet de limiter les différentes zones de conflits potentiels, et donc de pérenniser la fixation des prothèses concernées, tout en augmentant le confort du patient.

## Revendications

1. Cotyle pour prothèse de hanche comprenant :
- une cupule de soutien (4), destinée à être mise en place dans la cavité cotyloïdienne de l'aile iliaque de l'articulation à prothéser, et présentant une découpe partielle (8) s'étendant à partir de sa base (10) ;
- un insert de frottement (5), notamment réalisé en polyéthylène, destiné :
• à être inséré dans la cupule de soutien (4) tout en étant susceptible d'une complète mobilité au sein de celle-ci ;
• et à recevoir la tête (3) de l'élément fémoral (1) de ladite prothèse de manière autorétentive ;
- la cupule (4) étant de forme cylindro-hémisphérique et se prolongeante au niveau de son équateur par une portion sensiblement cylindrique, **caractérisé en ce que** la découpe partielle (8) qu'elle présente est évolutive et s'étend symétriquement d'une zone (I) située en amont de l'axe de révolution (7) de la cupule, c'est à dire en direction de son bord supérieur ou proximale, jusqu'à son bord inférieur ou distale (9).

2. Cotyle pour prothèse de hanche selon la revendication 1, ***caractérisé* en ce que** le départ de la découpe évolutive (8) est situé à quinze millimètres (I) de l'axe de révolution (7) de la cupule en direction de son coté supérieur ou proximale, et ce, quelle que soit la taille de la cupule mise en place.

3. Cotyle pour prothèse de hanche selon l'une des revendications 1 et 2, ***caractérisé* en ce que** la surface externe de la cupule (5) présente une zone en relief (11) s'étendant sensiblement sur au moins le tiers de sa hauteur à compter de la base (10), cette zone étant munie d'une pluralité de zones lisses (12, 13, 14).

4. Cotyle pour prothèse de hanche selon la revendication 3, ***caractérisé* en ce que** ces zones lisses sont au moins au nombre de trois, à savoir, l'une (12) située selon le plan de symétrie de la cupule à son extrémité supérieure, et les deux autres situées de part et d'autre symétriquement par rapport à la première.

5. Cotyle pour prothèse de hanche selon l'une des revendications 3 et 4, ***caractérisé* en ce que** la zone en relief (11) présente une surépaisseur progressive (20), destinée à éviter une discontinuité dans la répartition des contraintes à l'interface structure d'accroche- sphère vierge ou lisse, permettant de limiter les risques de protrusion et de rotation du cotyle, cette surépaisseur progressive présentant en outre une certaine discontinuité (21), destinée à favoriser la fixation secondaire du cotyle un fois implanté.

6. Cotyle pour prothèse de hanche selon la revendication 5, ***caractérisé* en ce que** la surépaisseur progressive (20) est constituées de saillies radiales.

7. Cotyle pour prothèse de hanche selon l'une des revendications 1 à 6, ***caractérisé* en ce que** l'arête périphérique externe (16) de l'insert (5) est rayonnée, de telle sorte à permettre audit insert de glisser sur les tissus périarticulaires (15) qui l'entourent, en lui conférant une mobilité accrue par rapport à ces tissus, et partant, de diminuer les frottements et les douleurs en résultant.

8. Cotyle pour prothèse de hanche selon l'une des revendications 1 à 7, ***caractérisé* en ce que** l'arête interne (17) de l'insert (5) est chanfreinée, de telle sorte à augmenter le débattement articulaire, et à limiter le contact entre le col (2) de l'élément fémoral et l'insert (5) et ainsi limiter les risques de production de débris d'usure du matériau constitutif dudit insert.

## Claims

1. Acetabular element for hip prosthesis, comprising:
- a support cup (4) intended to be placed in the acetabular cavity of the iliac crest of the articulation which is to be fitted with a prosthesis, and having a partial cutting (8) extending from its base (10);
- a friction insert (5), in particular made of polyethylene, intended:
· to be inserted in the support cup (4) while being permitted completely mobility inside the latter;
· and to receive the head (3) of the femoral element (1) of said prosthesis in a self-retaining manner;
the cup (4) being of cylindro-hemispherical shape and being continued at its equator by a substantially cylindrical portion, **characterized in that** its partial cutting (8) is evolutive and extends symmetrically from a zone (I) situated upstream of the axis of revolution (7) of the cup, that is to say in the direction of its upper or proximal edge, as far as its lower or distal edge (9).

2. Acetabular element for hip prosthesis according to Claim 1, **characterized in that** the start of the evolutive cutting (8) is situated at fifteen millimetres (I) from the axis of revolution (7) of the cup in the direction of its upper or proximal side, irrespective of the size of the cup implanted.

3. Acetabular element for hip prosthesis according to either of Claims 1 and 2, **characterized in that** the outer surface of the cup (5) [sic] has a zone in relief (11) extending substantially over at least the third of its height starting from the base (10), this zone being provided with a plurality of smooth zones (12, 13, 14).

4. Acetabular element for hip prosthesis according to Claim 3, **characterized in that** these smooth zones are at least three in number, namely one (12) situated in the plane of symmetry of the cup at its upper end, and the two others situated on either side symmetrically in relation to the first one.

5. Acetabular element for hip prosthesis according to either of Claims 3 and 4, **characterized in that** the zone in relief (11) has a progressive increased thickness (20) intended to avoid discontinuity in the distribution of the stresses at the interface between fastening structure and blank or smooth sphere, making it possible to limit the risks of protrusion and rotation of the acetabular element, this progressive increased thickness also having a certain discontinuity (21) intended to promote the secondary fixation of the acetabular element once it has been implanted.

6. Acetabular element for hip prosthesis according to Claim 5, **characterized in that** the progressive increased thickness (20) is made up of radial projections.

7. Acetabular element for hip prosthesis according to one of Claims 1 to 6, **characterized in that** the outer peripheral ridge (16) of the insert (5) is curved-in in such a way as to allow said insert to slide on the periarticular tissues (15) which surround it, conferring on it an increased mobility in relation to these tissues, and consequently reducing the friction and the pain which results from said friction.

8. Acetabular element for hip prosthesis according to one of Claims 1 to 7, **characterized in that** the inner ridge (17) of the insert (5) is bevelled in such a way as to increase the articular clearance and limit the contact between the neck (2) of the femoral element and the insert (5) and thereby limit the risks of production of debris from wear of the material constituting said insert.

## Patentansprüche

1. Gelenkpfanne für eine Hüftprothese mit:
- einer Stütz-Schale (4), welche dazu bestimmt ist, in die Hüftpfannenausnehmung der Beckenschaufel des mit der Prothese zu versehenen Gelenkes eingesetzt zu werden, und die einen partiellen Freischnitt (8) aufweist, der sich von ihrer Basis (10) ausgehend erstreckt;
- einem Gleiteinsatz (5), insbesondere aus Polyethylen hergestellt, der dazu bestimmt ist:
• in die Stütz-Schale (4) eingesetzt zu werden, wobei er darin vollständig beweglich ist;
• und den Kopf.(3) des femoralen Elementes (1) der genannten Prothese auf selbstfixierende Weise aufzunehmen;
- wobei die Schale (4) eine zylindrisch-halbkugelförmige Form aufweist und im Bereich ihres Äquators durch einen im wesentlichen zylindrischen Abschnitt verlängert ist, **dadurch gekennzeichnet, daß** ihr partieller Freischnitt (8) sich stetig entwickelt und sich symmetrisch aus einem Bereich (I) oberhalb der Hauptachse (7) der Schale, das heißt in Richtung ihres superioren oder proximalen Randes, bis zu ihrem unteren oder distalen Rand (9), erstreckt.

2. Gelenkpfanne für eine Hüftprothese nach Anspruch 1, **dadurch gekennzeichnet, daß** der Beginn des sich stetig entwickelnden Freischnittes (8) fünfzehn Millimeter (I) von der Hauptachse (7) der Schale in Richtung ihrer superioren oder proximalen Seite angeordnet ist, und zwar unabhängig von der Größe der verwendeten Schale.

3. Gelenkpfanne für eine Hüftprothese nach einem der Ansprüche 1 und 2, **dadurch gekennzeichnet, daß** die Außenfläche der Schale (5) eine strukturierte Zone (11) aufweist, die sich im wesentlichen über mindestens ein Drittel ihrer Höhe ausgehend von ihrer Basis (10) erstreckt und mit einer Mehrzahl von glatten Zonen (12,13,14) versehen ist.

4. Gelenkpfanne für eine Hüftprothese nach Anspruch 3, **dadurch gekennzeichnet, daß** sie mindestens drei glatte Zonen aufweist, nämlich eine (12), die in der Symmetrieebene der Schale an ihrem oberen Ende angeordnet ist, und zwei andere, die zur einen und zur anderen Seite symmetrisch in bezug auf die erste angeordnet sind.

5. Gelenkpfanne für eine Hüftprothese nach einem der Ansprüche 3 und 4, **dadurch gekennzeichnet, daß** die strukturierte Zone (11) eine progressive Überdicke (20) aufweist, welche dazu bestimmt ist, Unregelmäßigkeiten in der Verteilung der Lasten an der Grenzfläche zwischen Haltestruktur und einfacher oder glatter Kugelfläche zu vermeiden, was das Begrenzen der Gefahren der Protrusion und Rotation der Gelenkpfanne ermöglicht, wobei diese progressive Überdicke ferner eine gewisse Unterbrechung (21) aufweist, welche zur Begünstigung der Sekundärfixierung der implantierten Gelenkpfanne bestimmt ist.

6. Gelenkpfanne für eine Hüftprothese nach Anspruch 5, **dadurch gekennzeichnet, daß** die progressive Überdicke (20) von radialen Vorsprüngen gebildet wird.

7. Gelenkpfanne für eine Hüftprothese nach einem der Ansprüche 1 bis 6, **dadurch gekennzeichnet, daß** die periphere Außenkante (16) des Einsatzes (5) gerundet ist, so daß sie dem Einsatz ermöglicht, über die ihn und das Gelenk umgebenden Gewebe (15) zu gleiten, und ihm eine gesteigerte Beweglichkeit in bezug auf diese Gelenke verleiht und dadurch die resultierenden Reibungen und Schmerzen reduziert.

8. Gelenkpfanne für eine Hüftprothese nach einem der Ansprüche 1 bis 7, **dadurch gekennzeichnet, daß** die Innenkante (17) des Einsatzes (5) gefast ist, so daß die Gelenkfreiheit gesteigert und der Kontakt zwischen dem Hals (2) des femoralen Elements und dem Einsatz (5) begrenzt ist, um die Gefahren der Erzeugung von Abnutzungsabrieb des den genannten Einsatz bildenden Materials zu begrenzen.
